# EUROPÄISCHE PATENTSCHRIFT

(11) **EP 3 034 039 B1**
(45) Veröffentlichungstag und Bekanntmachung des Hinweises auf die Patenterteilung: **17.05.2017**
(21) Anmeldenummer: 15196454.1
(22) Anmeldetag: 26.11.2015
(51) Int. Cl.: A61F 2/18

(54) **GEHÖRKNÖCHELCHENPROTHESE MIT SPIKES**
AUDITORY OSSICLES PROSTHESIS WITH SPIKES
PROTHESE D'OSSELET A POINTE

(30) Priorität: 19.12.2014 DE 102014119224
(43) Veröffentlichungstag der Anmeldung: 22.06.2016
(73) Patentinhaber: Heinz Kurz GmbH Medizintechnik, 72144 Dusslingen (DE)
(72) Erfinder: Babu, Seilesh, Novi, MI Michigan 48374 (US); Steinhardt, Uwe, 72145 Hirrlingen (DE)
(74) Vertreter: Kohler Schmid Möbus Patentanwälte

(56) Entgegenhaltungen:
- EP-A1- 2 803 334
- DE-B3-102013 103 484
- US-A1- 2013 053 957

## Beschreibung

Die Erfindung betrifft eine aktive oder passive Gehörknöchelchenprothese, die zum Ersatz oder Überbrücken mindestens eines Elements der menschlichen Gehörknöchelchenkette ausgebildet ist, mit einem Schall übertragenden länglichen Prothesenkörper, welcher an seinem einen Ende ein erstes Ankoppelelement aufweist, das als eine Kopfplatte zur Anlage der Prothese am Trommelfell oder als ein Clip zur mechanischen Verbindung mit einem Glied der Gehörknöchelchenkette, insbesondere mit dem Ambossfortsatz oder mit dem Hammergriff, oder als ein Anschlussstück zur Schall leitenden Verbindung mit einem Aktor-Endstück eines aktiven Hör-Implantats gestaltet ist, wobei der längliche Prothesenkörper an seinem anderen Ende ein für eine mechanische Verbindung der Prothese mit einem Steigbügel oder mit einem Steigbügel-Köpfchen (= "caput") als seitlich mehrfach geschlitzte Glocke oder als Clip mit mehreren, jeweils alternierenden seitlichen Zungen und Schlitzen ausgebildetes zweites Ankoppelelement trägt, welches eine Zugangsöffnung in einen Aufnahmeraum mit einer Innenfläche in axialer Fortsetzung des länglichen Prothesenkörpers aufweist, die von einem umlaufenden, mehrfach unterbrochenen Außenrand umgrenzt ist. Eine solche Gehörknöchelchenprothese ist beispielsweise bekannt aus DE 10 2013 103 484 B3.

Im Bereich aktiver Hörimplantate sind ähnliche Vorrichtungen etwa in US 6,537,199 B1 oder in DE 10 2010 046 457 B3 beschrieben.

Bei passiven Gehörknöchelchenprothesen finden sich derartige Anordnungen mit unterschiedlich aufgebauten ersten und zweiten Ankoppelelementen zum Beispiel in US-A 5,514,177, in
WO 98/16175 A1, in EP 1 181 907 B1, in DE 10 2008 015 117 B3 oder etwa in DE 10 2009 016 468 B3.

Das menschliche Mittelohr mit seinen Gehörknöchelchen hat die Funktion, die über den äußeren Gehörgang auf das Trommelfell auftreffenden Schallwellen auf das mit Flüssigkeit gefüllte Innenohr zu übertragen. Die drei Gehörknöchelchen sind der am Trommelfell befestigte Hammer (lat. malleus), der Steigbügel (lat. stapes), der über seine Fußplatte (lat. basis stapedis) mit dem Innenohr verbunden ist, und der Amboss (lat. incus), der sich zwischen dem Hammer und dem Steigbügel befindet und mit diesen gelenkig verbunden ist. Beispielsweise die chronische Mittelohrentzündung ist eine Erkrankung des menschlichen Felsenbeins (= Knochen, in dem das gesamte Ohr sitzt), bei der es auf pathologisch-aggressive Art zu Abbauprozessen an der Gehörknöchelchenkette kommen kann. Dadurch wird das Schallsignal nicht oder nur unvollkommen zum Innenohr übertragen, was zur Schallleitungsschwerhörigkeit führt.

Hör-Implantate werden verwendet, um bei ganz oder teilweise fehlenden oder geschädigten Gehörknöchelchen des menschlichen Mittelohrs den auf die Ohrmuschel treffenden Schall bzw. ein entsprechendes Schallsignal zum Innenohr zu übertragen. Man unterscheidet dabei zwischen passiven Gehörknöchelchenprothesen einerseits, die Teile der Gehörknöchelchenkette physisch ersetzen, wobei die Schallleitung "passiv", also ohne Zuhilfenahme von energiebetriebenen Hilfsmitteln erfolgt, und aktiven Hör-Implantaten andererseits, die den Schallsignalen entsprechende energiebetriebene Signale aus einem meist elektronischen Verstärker eines extern oder auch intern angebrachten Hörgeräts mittels eines im Mittelohr implantierten Aktors empfangen, dort durch mechanische Bewegung wieder in akustische Schwingungen umsetzen und von einem vibrierenden Aktor-Endstück über ein geeignetes Verbindungselement zum Innenohr übertragen.

Passive Gehörknöchelchenprothesen dienen der Verbesserung der Schallübertragung bei unterschiedlichen pathologischen Befunden. Sie werden eingesetzt, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung zwischen dem Trommelfell und dem Innenohr nur begrenzt ermöglicht, weil sie die natürlichen anatomischen Ausbildungen der Gehörknöchelchenkette und die Mittelohrmechanik mit ihren feinen Strukturen nur sehr eingeschränkt ersetzen können.

Drei besonders häufig verwendete Arten von Gehörknöchelchenprothesen sind die Steigbügel-Prothesen, die Partial-Prothesen und die Total-Prothesen. Steigbügel-Prothesen (= Stapes-Prothesen) werden am Amboss fixiert und ragen über einen Stempel (= Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte. Die vorliegende Erfindung beschäftigt sich ausschließlich mit Partial-Prothesen.

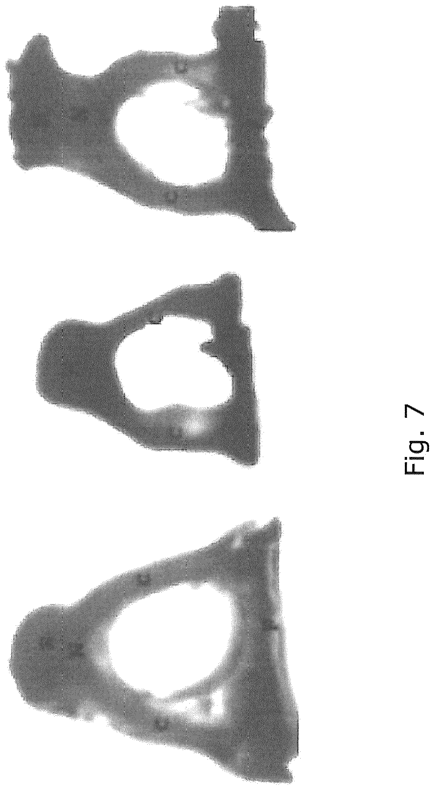

Wie aus den drei - stark vergrößerten - Aufnahmen von mehr oder weniger pathologischen menschlichen Steigbügel-Knochen in Fig. 7 deutlich wird, sind die anatomischen Unterschiede in der Gestalt und der absoluten Größe sowie auch in der jeweiligen Detail-Situation gerade im Bereich des Steigbügel-Köpfchens, wo ja die oben beschriebenen Partial-Prothesen mit ihrem zweiten Ankoppelelement angekoppelt werden sollen, drastisch unterschiedlich. Eine wirklich optimale Ankopplung an dieser Position würde daher für jeden Patienten eine ganz individuelle Formgebung des jeweils verwendeten Ankoppelelements am Implantat erfordern, was natürlich mit einem Aufwand in vernünftigen Grenzen nicht zu leisten ist.

Aufgabe der vorliegenden Erfindung ist es demgegenüber, eine gattungsgemäße - aktive oder passive - Gehörknöchelchenprothese der eingangs beschriebenen Art möglichst kostengünstig und mit simplen technischen Mitteln dahin gehend zu verbessern, dass die oben beschriebenen Vorteile der bekannten Partial-Prothesen für die Handhabung im Bereich des Mittelohres sowie im Ergebnis die damit erzielbare Verbesserung der Schallleitung beibehalten bleiben, wobei jedoch nach der Implantation auch für sehr lange Zeiträume ein verkipp-, verrutsch- und wackelsicherer Sitz der Prothese auf dem oberen Bereich des Steigbügel-Köpfchens garantiert werden soll und wobei auch auf einfache Weise weitere Freiheitsgrade zur individuellen Anpassung an die anatomischen Besonderheiten des einzelnen Patienten hinsichtlich der Form, Größe und Lage seines Steigbügel-Knöchelchens eröffnet werden.

Erfindungsgemäß wird diese Aufgabe ebenso überraschend einfach wie wirkungsvoll dadurch gelöst, dass das zweite Ankoppelelement am Außenrand der Innenfläche des Aufnahmeraums mindestens zwei über den Umfang des Außenrands verteilt angeordnete Spikes aufweist, die sich in einer Richtung parallel zur axialen Fortsetzung des länglichen Prothesenkörpers erstrecken und im implantierten Zustand der Gehörknöchelchenprothese in den Steigbügel, insbesondere in das Steigbügel-Köpfchen eingreifen um dort einen sicheren Halt des zweiten Ankoppelelements zu bewirken.

Durch das Eingreifen der Spikes in die Oberfläche des Steigbügel-Köpfchens wird ein auch für sehr lange Zeiträume im implantierten Zustand ein verrutsch-sicherer und positions-stabiler Sitz der Gehörknöchelchenprothese am Steigbügel ermöglicht, indem immer ein ausreichend starker mechanischer Kontakt zwischen dem zweiten Ankoppelelement und dem Steigbügel-Köpfchen garantiert ist. Außerdem wird auf diese Weise auch eine verkipp- und wackelsichere Seitenstabilisierung des zweiten Ankoppelelements in seiner End-Position erreicht.

Aufgrund des durch die erfindungsgemäß vorgesehenen Spikes auf einfache Weise sichergestellten, besonders dauerhaften mechanischen Sitzes der Prothese können die übrigen geometrischen Formgebungen der Prothese in relativ großen Grenzen frei gewählt und auf die individuellen Bedürfnisse und anatomischen Gegebenheiten beim einzelnen Patienten exakt angepasst und abgestimmt werden.

Bei Verwendung einer geschlitzten Glocke als zweites Ankoppelelement haben die Schlitze an der Glocke den Vorteil, dass die Prothese selbst dann über den Steigbügel gestülpt werden kann, wenn der oberste Teil des Steigbügels fehlen würde. Eine Variante von Weiterbildungen dieser Ausführungsform zeichnet sich dadurch aus, dass die Glocke eine rund gewölbte Glockenhaube aufweist. In vielen Fällen erweisen sich aber auch Weiterbildungen als günstig, bei welchen die Glocke eine abgeflachte und/oder von oben her eingedellte Glockenhaube aufweist. Dies hat zum Vorteil, dass es in axialer Verlängerung des Schaftes immer zu einem Kontakt kommt, so dass auch die Länge der Prothese eindeutig spezifiziert werden kann.

Eine alternative Klasse von Ausführungsformen zeichnet sich dadurch aus, dass das zweite Ankoppelelement als Clip mit mehreren, jeweils alternierenden seitlichen Zungen und Schlitzen ausgeführt ist. Diese Art von Ankoppelelementen wird häufig in Fällen eingesetzt, wo intraoperativ eine besonders hohe Stabilität gefordert wird, so dass der Chirurg eine einfache Anwendung hinsichtlich der Technik auffindet.

Bei besonders einfachen Ausführungsformen der erfindungsgemäßen Gehörknöchelchenprothese können genau zwei Spikes vorgesehen sein, die sich am Außenrand um 180° um den Umfang versetzt gegenüberliegen.

Eine noch höhere Stabilität des Sitzes der implantierten Gehörknöchelchenprothese ist bei alternativen Ausführungsformen zu erwarten, die sich dadurch auszeichnen, dass genau drei Spikes vorgesehen sind, die am Außenrand jeweils um 120° um den Umfang versetzt gegeneinander angeordnet sind und nach der Implantation gewissermaßen eine "Dreipunkt-Auflage" des zweiten Ankoppelelements auf dem Steigbügel-Köpfchen bewirken.

Ganz besonders hoch ist die mechanische Verankerung des zweiten Ankoppelelements der Gehörknöchelchenprothese und damit die Lage-Stabilität ihres post-operativen Sitzes auf dem Steigbügel-Köpfchen im Mittelohr, wenn genau vier Spikes vorgesehen sind, die am Außenrand jeweils um 90° um den Umfang versetzt gegeneinander angeordnet sind und die sich paarweise gegenüberliegen, so dass die gesamte Anordnung eine hohe Symmetrie aufweist.

Besonders bevorzugt ist auch eine Klasse von Ausführungsformen der erfindungsgemäßen Gehörknöchelchenprothese, bei welcher die Spikes Dreieck-förmig gestaltet sind, wobei eine Basis des Dreiecks fest mit dem Außenrand der Innenfläche des Aufnahmeraums verbunden ist und eine der Basis gegenüberliegende Spitze des Dreiecks von der Innenfläche weg ragt. Diese einfache Formgebung der Spikes ist zudem im Hinblick auf eine möglichst unkomplizierte Fertigbarkeit der Prothese von großem Vorteil, da sich ja die tatsächlichen Abmaße derartiger Details im Bereich von einigen Mikrometern bewegen.

Um eine erhöhte Flexibilität bzw. Variabilität der Prothese zu erreichen, kann bei bevorzugten Ausführungsformen der Erfindung der längliche Prothesenkörper mindestens ein Gelenk, insbesondere ein Kugelgelenk aufweisen, was zum Vorteil hat, dass die Prothese auch hydrostatische Kräfte kompensieren kann

Vorteilhaft im Hinblick auf eine besonders hohe postoperative Beweglichkeit der Prothese sind Weiterbildungen, bei denen eine Vielzahl von aneinander angrenzenden weiteren Drehelementen vorgesehen ist, vorzugsweise eine Kugelgelenkkette, die mit einem Hindurchschieben der Kugelkette durch die Gelenk-Aufnahme und nachfolgendes Entfernen der überstehenden obersten Kugeln auf einfache Weise eine Längenvariabilität der Prothese ermöglicht.

Andere bevorzugte Weiterbildungen dieser Ausführungsformen sehen vor, dass das Kugelgelenk eine an seinem dem zweiten Ankoppelelement zugewandten Ende des Prothesenkörpers angebrachte Kugel, eine die Kugel auf ihrer vom zweiten Ankoppelelement abgewandten Seite bedeckende Hülle sowie eine Vertiefung in der dem zweiten Ankoppelelement zugewandten Seite umfasst, welche für die Kugel als Gelenkpfanne wirkt. Diese Ausführungsformen führen dazu, dass das Kugelgelenk einer weichen Lagerung unterliegt und damit Dämpfungen aufnimmt.

Besonders bevorzugte Varianten dieser Weiterbildungen zeichnen sich dadurch aus, dass die Hülle des in den Prothesenkörper integrierten Kugelgelenks aus einem Kunststoff-Verguss, vorzugsweise aus einem Silikon-Verguss gebildet ist.

Zu den oben diskutierten Problemen von Partial-Prothesen im Mittelohr-Bereich tritt als nicht unbeachtliches Detailproblem hinzu, dass sich bei den gängigen Formen der bekannten und seit vielen Jahren eingesetzten Ankoppelelementen, etwa in Ausführungen als Stapes-Glocke oder auch bei Verwendung von Clips praktisch immer in Verlängerung des schaftförmigen Prothesenkörpers ein Hohlraum zwischen der Innenseite des Ankoppelelements und dem oberen Bereich des Steigbügel-Köpfchens ausbildet, weil letzteres in der Regel eine eher abgeflachte Form aufweist, während die an dieser Stelle üblichen Ankoppelelemente konkav gewölbt sind.

Eine Klasse von besonders vorteilhaften Ausführungsformen der erfindungsgemäßen Gehörknöchelchenprothese zeichnet sich deshalb dadurch aus, dass das zweite Ankoppelelement an der Innenfläche des Aufnahmeraums in axialer Fortsetzung des länglichen Prothesenkörpers einen vom Prothesenkörper weg in den Aufnahmeraum hinein ragenden Auffütterungsabschnitt aufweist, der im implantierten Zustand der Gehörknöchelchenprothese am Steigbügel, insbesondere am Steigbügel-Köpfchen anliegt und die Ausbildung eines Hohlraums zwischen dem Steigbügel und der Innenfläche des Aufnahmeraums in axialer Fortsetzung des länglichen Prothesenkörpers verhindert oder minimiert. Nach der Implantation wird eine Hohlraumbildung in axialer Verlängerung des Prothesenkörpers zwischen der Innenseite des Ankoppelelements und dem oberen Bereich des Steigbügel-Köpfchens sicher vermieden, wodurch sich die Schallleitung verbessert, weil kein Hohlraum als Schallhindernis mehr dazwischentritt. Außerdem werden weitere Freiheitsgrade zur individuellen Anpassung an die anatomischen Besonderheiten des einzelnen Patienten hinsichtlich der Form, Größe und Lage seines Steigbügel-Knöchelchens eröffnet.

Bei vielen Weiterbildungen dieser Klasse von Ausführungsformen der Erfindung wird in der Regel der Auffütterungsabschnitt kugelig oder ellipsoid gestaltet und symmetrisch zur verlängerten Achse des länglichen Prothesenkörpers angeordnet sein, so dass es immer zu einem direkten Anlage-Kontakt am Steigbügel kommt.

Für spezielle Situationen und individuelle Ausformungen des Steigbügel-Knöchelchens beim Patienten sind aber auch andere Ausgestaltungen der erfindungsgemäßen Gehörknöchelchenprothese vorteilhaft:
Eine alternative Weiterbildung etwa sieht vor, dass der Auffütterungsabschnitt kegelförmig gestaltet und symmetrisch zur verlängerten Achse des länglichen Prothesenkörpers angeordnet ist,
wobei die Kegelspitze vom länglichen Prothesenkörpers weg in den Aufnahmeraum hinein ragt. Dies für zu einer sehr schallharten Verbindung, welche durch die punktartige Belastung seitlich fixiert wird.

Möglich sind aber auch Weiterbildungen, bei welchen der Auffütterungsabschnitt zylinderförmig gestaltet und symmetrisch zur verlängerten Achse des länglichen Prothesenkörpers angeordnet ist, wobei der Zylinder vom länglichen Prothesenkörpers weg in den Aufnahmeraum hinein ragt.

Bei anderen Weiterbildungen ist der Auffütterungsabschnitt stempelförmig gestaltet und symmetrisch zur verlängerten Achse des länglichen Prothesenkörpers angeordnet, wobei ein den Stempelkörper tragender Stempelschaft vom länglichen Prothesenkörpers weg in den Aufnahmeraum hinein ragt.

Diese Weiterbildungen können, je nach den individuellen Bedürfnissen des Patienten, insbesondere der genauen Formgebung des Steigbügel-Köpfchens wiederum in unterschiedlicher Weise ausgestaltet werden:
Bei einer Variante dieser Weiterbildungen weist der Stempelkörper eine in den Aufnahmeraum gerichtete konkave Auflagefläche auf, welche auch dann direkt greift, falls das Steigbügelköpfchen eine konvexe Form haben sollte.

Eine andere Variante zeichnet sich dadurch aus, dass der Stempelkörper eine in den Aufnahmeraum gerichtete ebene Auflagefläche aufweist.

In anders gelagerten Fällen kann aber auch eine Variante nützlich sein, bei der der Stempelkörper eine in den Aufnahmeraum gerichtete konvexe Auflagefläche aufweist, welche auch dann direkt greift, falls das Steigbügelköpfchen eine konkave Form haben sollte.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein:
In vielen Ausführungsformen der Erfindung wird das erste Ankoppelelement eine zur Anlage am Trommelfell ausgebildete Kopfplatte umfassen. Bei anderen Ausführungsformen kann beispielsweise die Prothese einerseits am Ambossfortsatz oder am Hammergriff befestigt sein. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Neben postoperativen Positionsverschiebungen ergibt sich nach der Implantation von Gehörknöchelchenprothesen häufig noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel.

Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen. Ein als Kopfplatte ausgeführtes erstes Ankoppelelement der erfindungsgemäßen Gehörknöchelchenprothese sollte grundsätzlich eine wachstumsfördernde Beschichtung aufweisen.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Ganz besonders vorteilhaft sind Ausführungsformen der Erfindung, bei denen das zweite Ankoppelelement ganz oder teilweise aus Titan oder einem Material mit Formgedächtnis (= memory effect) und/oder mit superelastischen Eigenschaften, insbesondere aus Nitinol hergestellt ist. Die Verwendung derartiger Materialien ist auf dem Gebiet der Gehörknöchelchenprothesen an sich bekannt, erweist sich aber gerade im Zusammenhang mit der vorliegenden Erfindung als besonders wirkungsvoll.

Vorteilhaft im Hinblick auf die oben erwähnte postoperative Lageanpassung sind Ausführungsformen der Erfindung, bei denen die gesamte Prothese oder andere Teile davon, insbesondere auch das erste Ankoppelelement, aus einem Material mit Formgedächtnis (= memory effect) oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Möglich sind aber auch Ausführungsformen der Erfindung, bei denen die gesamte Prothese oder Teile davon aus biokompatiblen Kunststoffen, insbesondere Silicon, Polytetrafluorethylen (PTFE) oder Faserverbundwerkstoffen hergestellt sind. Mit diesen Materialien können post-operative Abstoßungsreaktionen in den meisten Fällen ebenfalls verhindert werden.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können jeweils einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1a: eine schematische räumliche Darstellung von schräg oben auf eine erste Ausführungsform der erfindungsgemäßen Gehörknöchelchenprothese mit einer Trommelfell-Kopfplatte als erstem Ankoppelelemente sowie einem clip-förmigen zweiten Ankoppelelement mit mehreren, jeweils alternierenden seitlichen Zungen und Schlitzen;
- Fig. 1b: die Ausführungsform gemäß Fig. 1a von unten mit Blickrichtung in den Aufnahmeraum des zweiten Ankoppelelements;
- Fig. 1c: die Ausführungsform gemäß Fig. 1a von der Seite;
- Fig. 2a: eine schematische räumliche Darstellung von schräg oben auf eine erste Ausführungsform der erfindungsgemäßen Gehörknöchelchenprothese mit einer Trommelfell-Kopfplatte als erstem Ankoppelelement sowie einer mehrfach geschlitzten Glocke als zweitem Ankoppelelement zur Auflage auf dem Steigbügel-Köpfchen;
- Fig. 2b: die Ausführungsform gemäß Fig. 2a schräg von unten mit Blick in den Aufnahmeraum des zweiten Ankoppelelements;
- Fig. 2c: die Ausführungsform gemäß Fig. 2a von der Seite;
- Fig. 3: eine längenvariable Ausführungsform der erfindungsgemäßen Gehörknöchelchenprothese mit Kugelkette als Prothesenkörper und 4 Spikes am glockenförmigen zweiten Ankoppelelement sowie mit in den Aufnahmeraum des zweiten Ankoppelelements hinein ragendem Auffütterungsabschnitt;
- Fig. 4: Ausführungsform mit Kugelgelenk im Prothesenkörper und mit Auffütterungsabschnitt sowie mit 3 Spikes im glockenförmigen zweiten Ankoppelelement;
- Fig. 5: eine schematische Schnittdarstellung einer Ausführungsform der erfindungsgemäßen Gehörknöchelchenprothese im Bereich eines als geschlitzte Glocke ausgeführten zweiten Ankoppelelements mit halbkugelig gestaltetem Auffütterungsabschnitt und mit in den Prothesenkörper endseitig integriertem Kugelgelenk, wobei der Auffütterungsabschnitt des zweiten Ankoppelelements als Gelenkpfanne aus gebildet ist;
- Fig. 6: eine Ausführungsform mit einem als Anschlussstück zur Schall leitenden Verbindung mit einem Aktor-Endstück eines aktiven Hör-Implantats ausgebildeten ersten Befestigungselement und einer mehrfach geschlitzten Glocke als zweitem Ankoppelelement, welches einen Auffütterungsabschnitt und zwei Spikes aufweist; und
- Fig. 7: drei fotografische Aufnahmen mit jeweils gleicher Vergrößerung von unterschiedlichen, teilweise pathologisch veränderten menschlichen Steigbügel-Knöchelchen mit insbesondere verschiedenen Geometrien des jeweiligen Steigbügel-Köpfchens.

Die in den Figuren der Zeichnung schematisch dargestellten - im Detail unterschiedlich gestalteten - Ausführungsformen der erfindungsgemäßen **Gehörknöcheichenprothese 10; 20; 30; 40; 50; 60** weisen am einen Ende jeweils ein **erstes Ankoppelelement 11; 21; 31; 41; 61** auf, welches der mechanischen Verbindung der Prothese mit einem Glied der Gehörknöchelchenkette dient und als Trommelfell-Kopfplatte 11; 21; 31; 41 zur Anlage am Trommelfell oder als ein Anschlussstück 61 zur Schall leitenden Verbindung mit einem Aktor-Endstück eines aktiven Hör-Implantats gestaltet ist. Am anderen Ende der Gehörknöchelchenprothese 10; 20; 30; 40; 50; 60 sitzt jeweils ein **zweites Ankoppelelement 12; 22; 32; 42; 52; 62** mit einer **Zugangsöffnung 14; 24** in einen **Aufnahmeraum 15; 25,** das für eine mechanische Verbindung der Prothese mit dem **Steigbügel,** insbesondere mit dem **Steigbügel-Köpfchen,** als seitlich mehrfach geschlitzte Glocke 22; 32; 42; 52; 62 oder als Clip 12 mit mehreren, jeweils alternierenden seitlichen **Zungen 12a** und **Schlitzen 12b** ausgebildet ist. Dazwischen ist ein die beiden Ankoppelelemente 11; 21; 31; 41; 61 bzw. 12; 22; 32; 42; 52; 62 Schall leitend miteinander verbindender **länglicher Prothesenkörpers 13; 23; 33; 43; 53; 63** in Form eines länglichen Schaftes angeordnet.

Der Aufnahmeraum 15; 25 weist eine Innenfläche in axialer Fortsetzung des länglichen Prothesenkörpers 13; 23; 33; 43; 53; 63 auf, die von einem umlaufenden, mehrfach unterbrochenen **Außenrand 18; 28** umgrenzt ist.

Erfindungsgemäß ist das zweite Ankoppelelement das zweite Ankoppelelement 12; 22; 32; 42; 52; 62 so gestaltet, dass es am Außenrand 18; 28 der Innenfläche des Aufnahmeraums 15; 25 mindestens zwei über den Umfang des Außenrands 18; 28 verteilt angeordnete **Spikes 19; 29; 29'; 29"** aufweist, die sich in einer Richtung parallel zur axialen Fortsetzung des länglichen Prothesenkörpers 13; 23; 33; 43; 53; 63 erstrecken und im implantierten Zustand der Gehörknöchelchenprothese 10; 20; 30; 40; 50; 60 in den Steigbügel, insbesondere in das Steigbügel-Köpfchen eingreifen um dort einen sicheren Halt des zweiten Ankoppelelements 12; 22; 32; 42; 52; 52 zu bewirken.

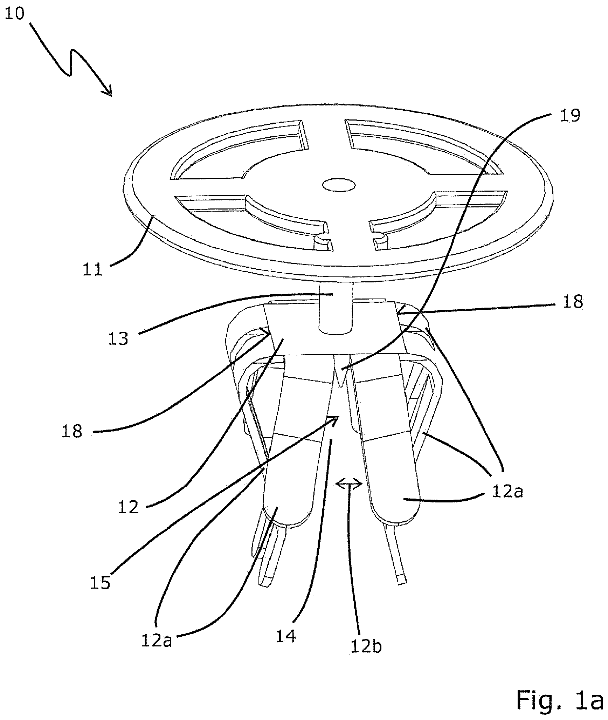

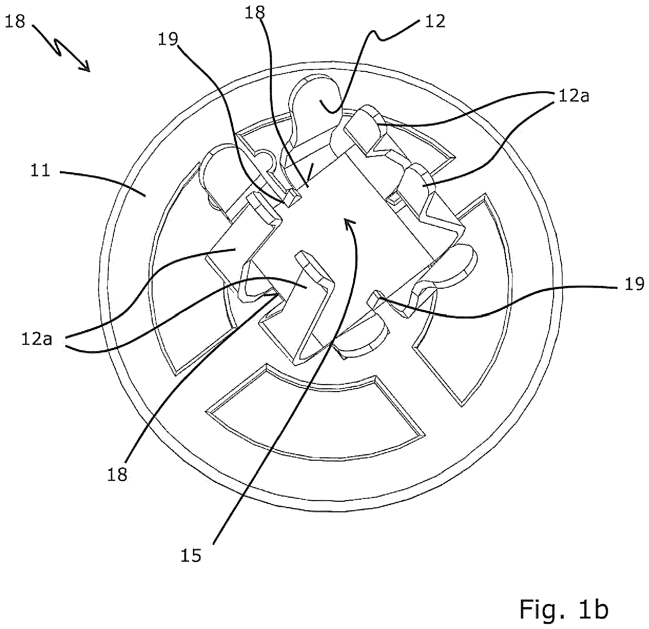

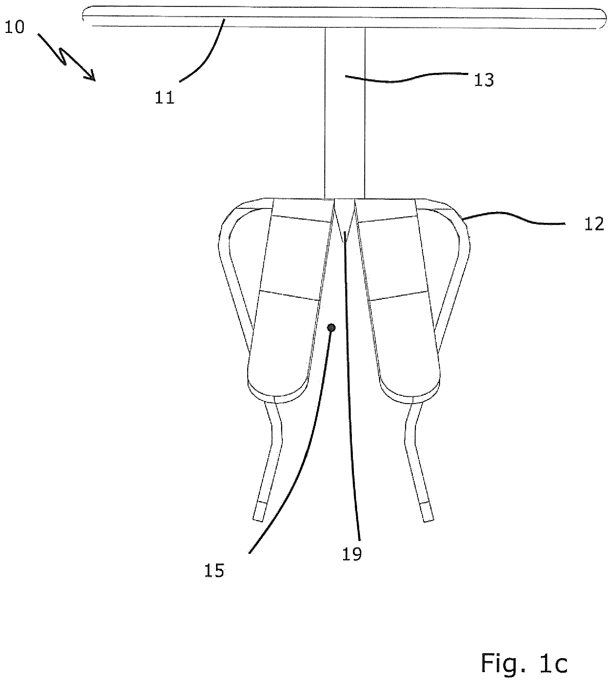

Bei sämtlichen in den Figuren 1a bis 2c und 6 dargestellten Ausführungsformen sind beim zweiten Ankoppelelement 12; 22; 62 am Außenrand 18; 28 der Innenfläche des Aufnahmeraums 15; 25 jeweils genau zwei Spikes 19; 29 vorgesehen, die sich um 180° um den Umfang versetzt gegenüberliegen.

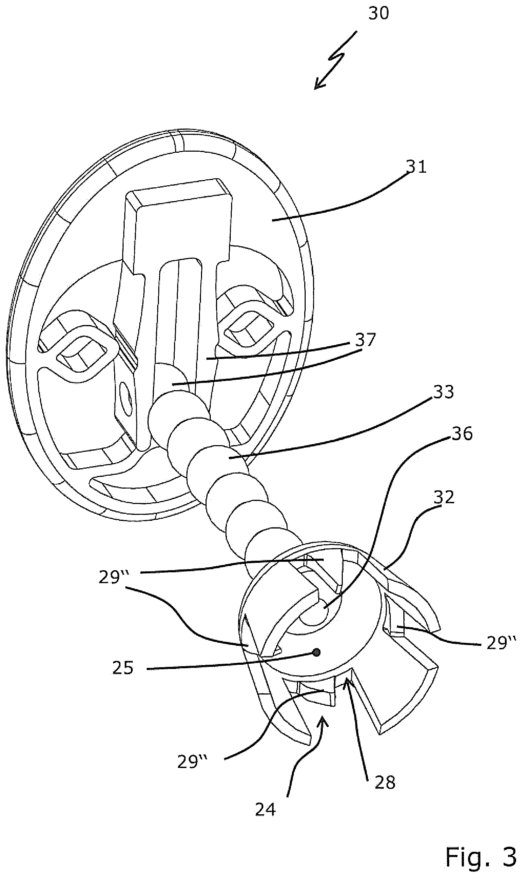

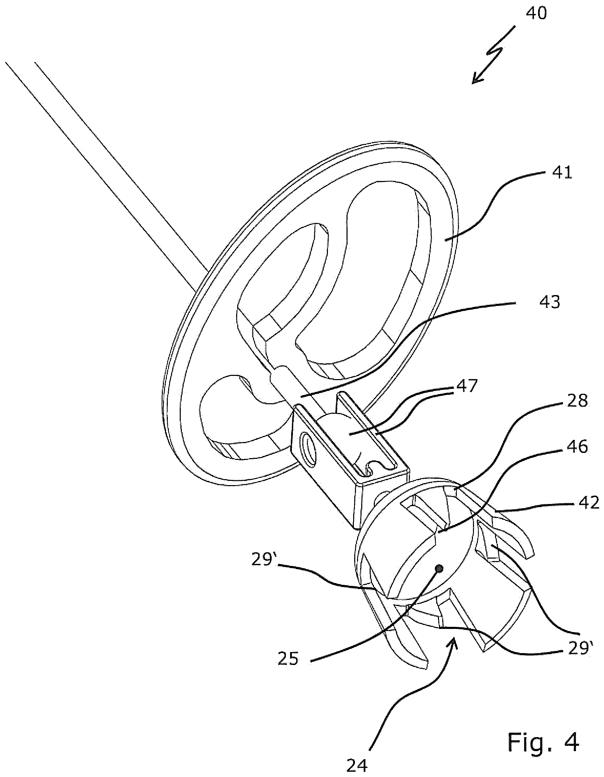

Die Ausführungsform gemäß Fig. 4 zeichnet sich durch genau drei Spikes 29' aus, die am Außenrand 28 jeweils um 120° um den Umfang versetzt gegeneinander angeordnet sind. Bei der Ausführungsform gemäß Fig. 3 sind genau vier Spikes 29" vorgesehen, die am Außenrand 28 jeweils um 90° um den Umfang versetzt gegeneinander angeordnet sind und sich paarweise gegenüberliegen.

Vorzugsweise sind die Spikes 19; 29; 29'; 29" Dreieck-förmig gestaltet, wobei eine Basis des Dreiecks fest mit dem Außenrand 18; 28 der Innenfläche des Aufnahmeraums 15; 25 verbunden ist und eine der Basis gegenüberliegende Spitze des Dreiecks von der Innenfläche weg ragt.

Bei der in den Figuren 1a bis 1c gezeigten Ausführungsform der erfindungsgemäßen Gehörknöchelchenprothese 10 ist das erste Ankoppelelement 11 in Form einer Kopfplatte zur Anlage am Trommelfell ausgebildet. Das zweite Ankoppelelement 12 an dem der Kopfplatte entgegen gesetzten Ende des länglichen Prothesenkörpers 13 ist in diesem Ausführungsbeispiel als Clip mit mehreren, jeweils alternierenden seitlichen Zungen 12a und Schlitzen 12b zwischen den Zungen 12a ausgeführt.

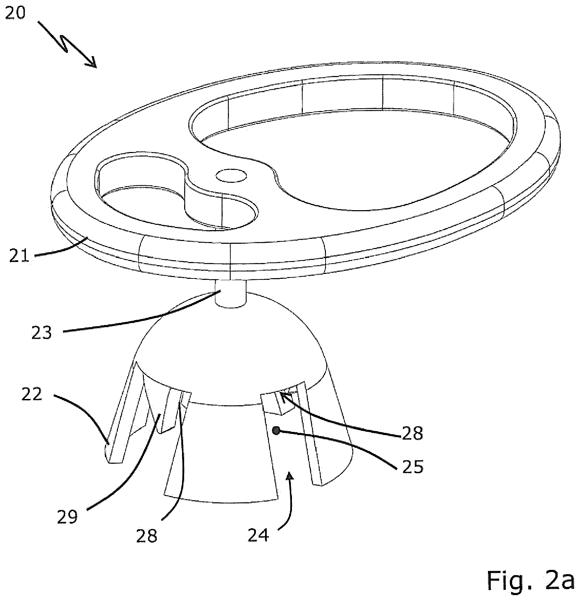

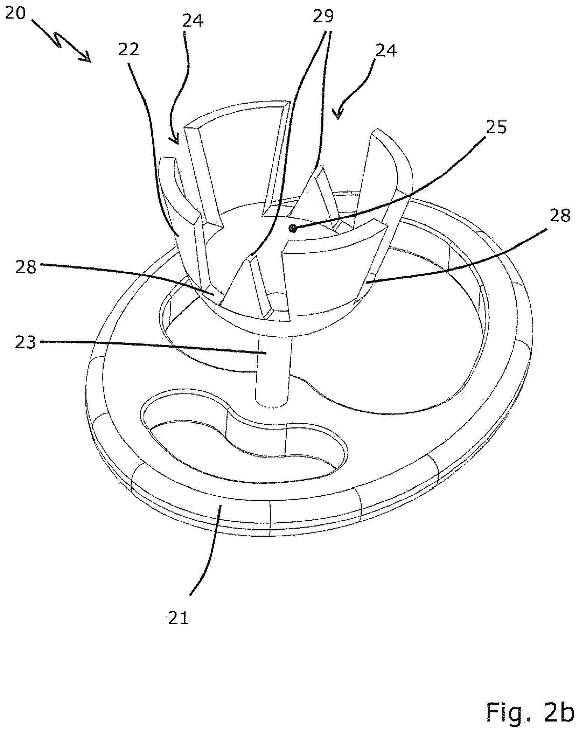

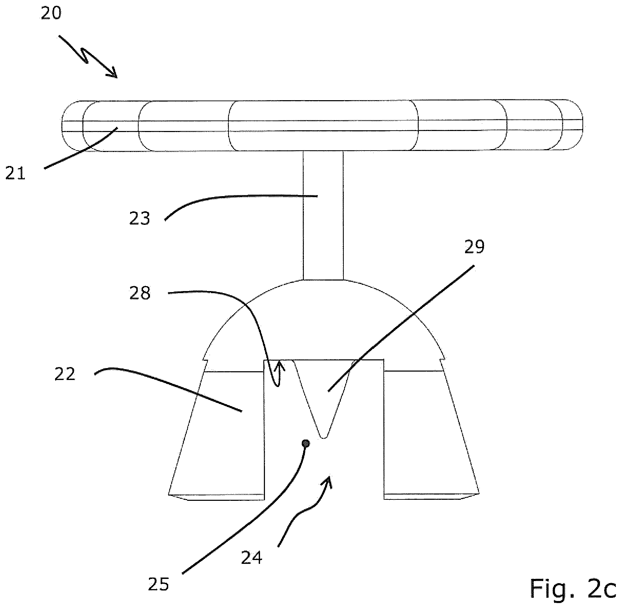

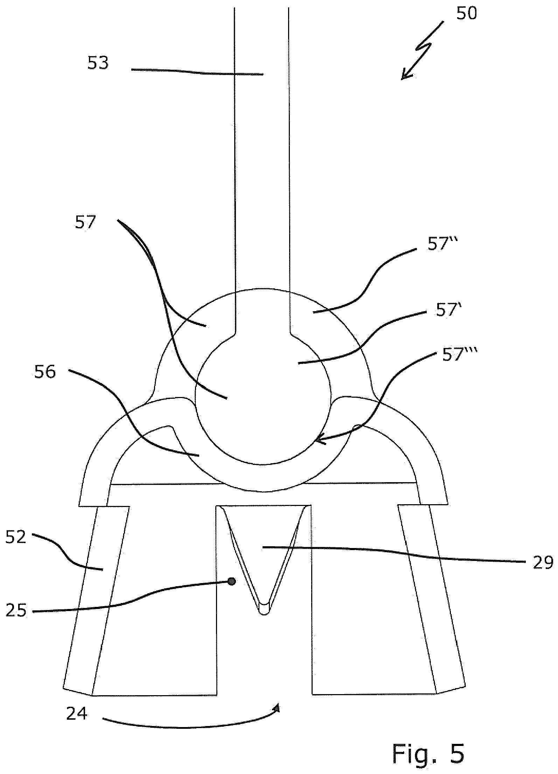

Bei den Ausführungsformen der Figuren 2a bis 2c ist das erste Ankoppelelement 21 wiederum als Kopfplatte zur Anlage am Trommelfell ausgebildet. Das zweite Ankoppelelement 22 ist als mehrfach geschlitzte Glocke zur Auflage auf dem Steigbügel-Köpfchen gestaltet und mit der Glockenhaube an dem dem ersten Ankoppelelement 21 gegenüber liegenden Ende des länglichen Prothesenkörpers 23 befestigt. Letzteres trifft auch für die in den Figuren 3 bis 6 dargestellten Ausführungsformen zu. Dabei weisen die glockenförmigen Ankoppelelemente 22; 32; 42; 52; 62 in den Figuren 2a bis 4 und 6 jeweils eine rund gewölbte Glockenhaube auf, das glockenförmige Ankoppelelement 52 in Figur 5 eine oben eingedellte Glockenhaube. Bei in der Zeichnung nicht dargestellten Ausführungsformen der Erfindung kann das glockenförmige Ankoppelelement auch eine abgeflachte Glockenhaube aufweisen.

Die Ausführungsformen gemäß den Figuren 3 bis 6 sind dadurch gekennzeichnet, dass das zweite Ankoppelelement 32; 42; 52; 62 an der Innenfläche des jeweiligen Aufnahmeraums 25 in axialer Fortsetzung des länglichen Prothesenkörpers 33; 43; 53; 63 einen vom länglichen Prothesenkörper 33; 43; 53; 63 weg in den Aufnahmeraum 25 hinein ragenden **Auffütterungsabschnitt 36; 46; 56; 66** aufweist, der im implantierten Zustand der Gehörknöchelchenprothese 30; 40; 50; 60 am Steigbügel, insbesondere am Steigbügel-Köpfchen anliegt und die Ausbildung eines Hohlraums zwischen dem Steigbügel und der Innenfläche des Aufnahmeraums 25 in axialer Fortsetzung des länglichen Prothesenkörpers 33; 43; 53; 63 verhindert oder minimiert.

Der Auffütterungsabschnitt 36; 46; 56; 66 ist bei den gezeigten Ausführungsformen der Figuren 3 bis 6 ist kugelig oder ellipsoid gestaltet und symmetrisch zur verlängerten Achse des länglichen Prothesenkörpers 33; 43; 53; 63 angeordnet.

Die in den Figuren 3 bis 5 dargestellten Ausführungsformen zeichnen sich zudem dadurch aus, dass der längliche Prothesenkörper 33; 43; 53 mindestens ein **Gelenk 37; 47; 57** aufweist, insbesondere ein Kugelgelenk.

Während das Kugelgelenk 37 der Gehörknöchelchenprothese 30 gemäß Fig. 3 einen als Kugelkette ausgestalteten länglichen Prothesenkörper 33 aufweist, ist das Kugelgelenk 47 bei der Gehörknöchelchenprothese 40 in Fig. 4 in den schaftförmigen länglichen Prothesenkörper 43 integriert. Die Kugelkette des Kugelgelenks 37 in Fig. 3 kann durch einen Aufnahmeabschnitt in dem als TrommelfellKopfplatte ausgeführten ersten Ankoppelelement 31 hindurchgeführt und auf die jeweils aktuell erforderliche Länge abgeschnitten werden. Die übrig bleibende oberste Kugel des länglichen Prothesenkörpers 33 bildet dann zusammen mit dem Aufnahmeabschnitt das Kugelgelenk 37.

Bei der Ausführungsform nach Fig. 5 umfasst das Kugelgelenk 57 eine an seinem dem zweiten Ankoppelelement 52 zugewandten Ende des länglichen Prothesenkörpers 53 angebrachte **Kugel 57',** eine die Kugel 57' auf ihrer vom zweiten Ankoppelelement 52 abgewandten Seite bedeckende **Hülle 57"** sowie eine in die Außenfläche der Glockenhaube des zweiten Ankoppelelements 52 eingedellte **Vertiefung 57"'**, welche für die Kugel 57' als Gelenkpfanne wirkt. Die Hülle 57" ist aus einem Kunststoff-Verguss, vorzugsweise aus einem Silikon-Verguss gebildet. Die dem Aufnahmeraum 25 zugewandte Innenseite der Glockenhaube bildet aufgrund der oben beschriebenen Eindellung einen halbkugeligen Auffütterungsabschnitt 56.

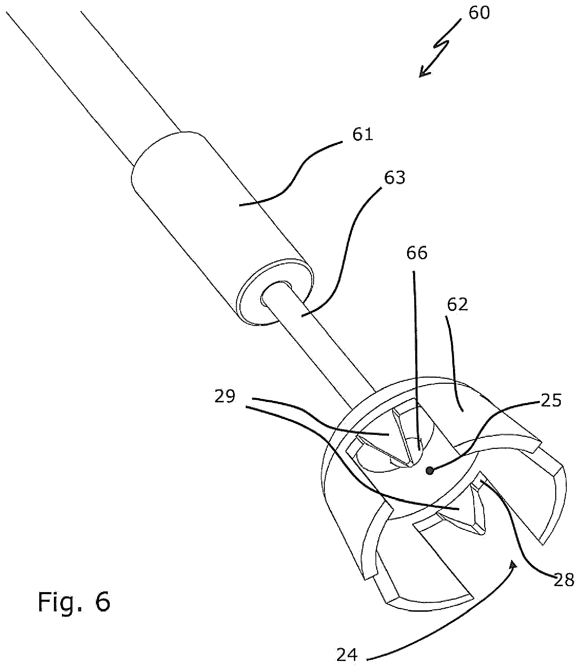

Bei der Ausführungsform gemäß Fig. 6 ist das erste Ankoppelelement 61 als Anschlussstück zur Schall leitenden Verbindung mit einem Aktor-Endstück eines - in der Zeichnung nicht näher dargestellten - aktiven Hör-Implantats gestaltet, während das zweite Ankoppelelement 62 wiederum die Form einer mehrfach geschlitzten Glocke aufweist.

In Fig. 7 schließlich sind drei photographische Aufnahmen von menschlichen Steigbügel-Knöchelchen mit jeweils gleicher Vergrößerung gezeigt. Man erkennt erhebliche Unterschiede in der geometrischen Form des Steigbügels, insbesondere im Bereich des Steigbügel-Köpfchens, aber auch deutlich verschiedene Größen des jeweiligen Steigbügel-Knöchelchens sowie seiner Details, die teilweise durch pathologische Prozesse oder einfach nur durch die natürlichen individuellen Abweichung von einem Patienten zum anderen hervorgerufen werden. Hier sind die entscheidenden Vorteile verdeutlicht, welche die Variationsmöglichkeiten bei einer erfindungsgemäß modifizierten Gehörknöchelchenprothese im Gegensatz zu einer herkömmlichen Prothese nach dem Stand der Technik im Hinblick auf eine individuelle Problemlösung bieten. Aufgrund der erfindungsgemäß vorgesehenen Spikes am zweiten Ankoppelelement sowie den diversen Ausgestaltungsmöglichkeiten des optional zusätzlichen Auffütterungsabschnitts kann gerade im Bereich des Steigbügel-Köpfchens eine optimale Schall-Weiterleitung sichergestellt werden.

## Patentansprüche

1. Gehörknöchelchenprothese (10; 20; 30; 40; 50; 60), die zum Ersatz oder Überbrücken mindestens eines Elements der menschlichen Gehörknöchelchenkette ausgebildet ist, mit einem Schall übertragenden länglichen Prothesenkörper (13; 23; 33; 43; 53; 63), welcher an seinem einen Ende ein erstes Ankoppelelement (11; 21; 31; 41; 61) aufweist, das als eine Kopfplatte (11; 21; 31; 41) zur Anlage der Prothese am Trommelfell oder als ein Clip zur mechanischen Verbindung mit einem Glied der Gehörknöchelchenkette oder als ein Anschlussstück (61) zur Schall leitenden Verbindung mit einem Aktor-Endstück eines aktiven Hör-Implantats gestaltet ist, wobei der längliche Prothesenkörper (13; 23; 33; 43; 53; 63) an seinem anderen Ende ein für eine mechanische Verbindung der Prothese mit einem Steigbügel oder mit einem Steigbügel-Köpfchen als Clip (12) mit mehreren, jeweils alternierenden seitlichen Zungen (12a) und Schlitzen (12b) oder als seitlich mehrfach geschlitzte Glocke (22; 32; 42; 52; 62) ausgebildetes zweites Ankoppelelement (12; 22; 32; 42; 52; 62) trägt, welches eine Zugangsöffnung (14; 24) in einen Aufnahmeraum (15; 25) mit einer Innenfläche in axialer Fortsetzung des länglichen Prothesenkörpers (13; 23; 33; 43; 53; 63) aufweist, die von einem umlaufenden, mehrfach unterbrochenen
Außenrand (18; 28) umgrenzt ist,
**dadurch gekennzeichnet,**
**dass** das zweite Ankoppelelement (12; 22; 32; 42; 52; 62) am Außenrand (18; 28) der Innenfläche des Aufnahmeraums (15; 25) mindestens zwei über den Umfang des Außenrands (18; 28) verteilt angeordnete Spikes (19; 29; 29'; 29") aufweist, die sich in einer Richtung parallel zur axialen Fortsetzung des länglichen Prothesenkörpers (13; 23; 33; 43; 53; 63) erstrecken und im implantierten Zustand der Gehörknöchelchenprothese (10; 20; 30; 40; 50; 60) in den Steigbügel eingreifen um dort einen sicheren Halt des zweiten Ankoppelelements (12; 22; 32; 42; 52; 62) zu bewirken.

2. Gehörknöchelchenprothese nach Anspruch 1, **dadurch gekennzeichnet, dass** genau zwei Spikes (19; 29) vorhanden sind, die sich am Außenrand (18; 28) um 180° um den Umfang versetzt gegenüberliegen.

3. Gehörknöchelchenprothese nach Anspruch 1, **dadurch gekennzeichnet, dass** genau drei Spikes (29`) vorhanden sind, die am Außenrand (28) jeweils um 120° um den Umfang versetzt gegeneinander angeordnet sind.

4. Gehörknöchelchenprothese nach Anspruch 1, **dadurch gekennzeichnet, dass** genau vier Spikes (29") vorhanden sind, die am Außenrand (28) jeweils um 90° um den Umfang versetzt gegeneinander angeordnet sind und sich paarweise gegenüberliegen.

5. Gehörknöchelchenprothese nach einem der vorhergehenden Ansprüche, **dadurch gekennzeichnet, dass** die Spikes (19; 29; 29'; 29") Dreieck-förmig gestaltet sind, wobei eine Basis des Dreiecks fest mit dem Außenrand (18; 28) der Innenfläche des Aufnahmeraums (15; 25) verbunden ist und eine der Basis gegenüberliegende Spitze des Dreiecks von der Innenfläche weg ragt.

6. Gehörknöchelchenprothese nach einem der vorhergehenden Ansprüche, **dadurch gekennzeichnet, dass** der längliche Prothesenkörper (33; 43; 53) mindestens ein Gelenk (37; 47; 57) aufweist.

7. Gehörknöchelchenprothese nach Anspruch 6, **dadurch gekennzeichnet, dass** das Gelenk (57) eine an seinem dem zweiten Ankoppelelement (52) zugewandten Ende des länglichen Prothesenkörpers (53) angebrachte Kugel (57'), eine die Kugel (57') auf ihrer vom zweiten Ankoppelelement (52) abgewandten Seite bedeckende Hülle (57") sowie eine Vertiefung (57''') in der dem zweiten Ankoppelelement (52) zugewandten Seite umfasst, welche für die Kugel (57') als Gelenkpfanne wirkt.

8. Gehörknöchelchenprothese nach Anspruch 7, **dadurch gekennzeichnet, dass** die Hülle (57") aus einem Kunststoff-Verguss gebildet ist.

9. Gehörknöchelchenprothese nach einem der vorhergehenden Ansprüche, **dadurch gekennzeichnet, dass** das zweite Ankoppelelement (32; 42; 52; 62) an der Innenfläche des Aufnahmeraums (25) in axialer Fortsetzung des länglichen Prothesenkörpers (33; 43; 53; 63) einen vom länglichen Prothesenkörper (33; 43; 53; 63) weg in den Aufnahmeraum (25) hinein ragenden Auffütterungsabschnitt (36; 46; 56; 66) aufweist, der im implantierten Zustand der Gehörknöchelchenprothese (30; 40; 50; 60) am Steigbügel anliegt und die Ausbildung eines Hohlraums zwischen dem Steigbügel und der Innenfläche des Aufnahmeraums (25) in axialer Fortsetzung des länglichen Prothesenkörpers (33; 43; 53; 63) verhindert oder minimiert.

10. Gehörknöchelchenprothese nach Anspruch 9, **dadurch gekennzeichnet, dass** der Auffütterungsabschnitt (36; 46; 56; 66) kugelig oder ellipsoid gestaltet und symmetrisch zur verlängerten Achse des länglichen Prothesenkörpers (33; 43; 53; 63) angeordnet ist.

11. Gehörknöchelchenprothese nach Anspruch 9, **dadurch gekennzeichnet, dass** der Auffütterungsabschnitt kegelförmig gestaltet und symmetrisch zur verlängerten Achse des länglichen Prothesenkörpers angeordnet ist, wobei eine Kegelspitze vom länglichen Prothesenkörpers weg in den Aufnahmeraum hinein ragt.

12. Gehörknöchelchenprothese nach Anspruch 9, **dadurch gekennzeichnet, dass** der Auffütterungsabschnitt zylinderförmig gestaltet und symmetrisch zur verlängerten Achse des länglichen Prothesenkörpers angeordnet ist, wobei ein Zylinder vom länglichen Prothesenkörpers weg in den Aufnahmeraum hinein ragt.

13. Gehörknöchelchenprothese nach Anspruch 9, **dadurch gekennzeichnet, dass** der Auffütterungsabschnitt stempelförmig gestaltet und symmetrisch zur verlängerten Achse des länglichen Prothesenkörpers angeordnet ist, wobei ein einen Stempelkörper tragender Stempelschaft vom länglichen Prothesenkörpers weg in den Aufnahmeraum hinein ragt.

14. Gehörknöchelchenprothese nach Anspruch 13, **dadurch gekennzeichnet, dass** der Stempelkörper eine in den Aufnahmeraum gerichtete konkave oder ebene Auflagefläche aufweist.

15. Gehörknöchelchenprothese nach Anspruch 13, **dadurch gekennzeichnet, dass** der Stempelkörper eine in den Aufnahmeraum gerichtete konvexe Auflagefläche aufweist.

## Claims

1. Auditory ossicle prosthesis (10; 20; 30; 40; 50; 60) designed to replace or bridge at least one element of the human ossicular chain, comprising an elongated sound-transmitting prosthesis body (13; 23; 33; 43; 53; 63) provided at one end with a first coupling element (11; 21; 31; 41; 61) designed as a top plate (11; 21; 31; 41) for bringing the prosthesis to bear against the eardrum, as a clip for a mechanical connection to an element of the ossicular chain or as a connecting piece (61) for a sound-conducting connection to an actuator end piece of an active hearing implant, the elongated prosthesis body (13; 23; 33; 43; 53; 63) being provided at its other end with a second coupling element (12; 22; 32; 42; 52; 62) designed as a clip (12) having a plurality of alternating lateral prongs (12a) and slots (12b) or as a bell (22; 32; 42; 52; 62) having a plurality of lateral slots for a mechanical connection of the prosthesis to the stapes or to the head of stapes, said second coupling element having an access opening (14; 24) to a receiving space (15; 25) having an inner surface forming an axial continuation of the elongated prosthesis body (13; 23; 33; 43; 53; 63) and delimited by a circumferential outer edge (18; 28) having a plurality of interruptions, **characterised in that** the second coupling element (12; 22; 32; 42; 52; 62) is provided on the outer edge (18; 28) of the inner surface of the receiving space (15; 25) with at least two spikes (19; 29; 29'; 29") distributed around the circumference of the outer edge (18; 28), extending in a direction parallel to the axial continuation of the elongated prosthesis body (13; 23; 33; 43; 53; 63) and engaging in the implanted state of the auditory ossicle prosthesis (10; 20; 30; 40; 50; 60) in the stapes so as to hold the second coupling element (12; 22; 32; 42; 52; 62) securely in place.

2. Auditory ossicle prosthesis according to claim 1, **characterised by** precisely two spikes (19; 29) arranged opposite one another on the outer edge (18; 28) in such a manner that they are offset by 180° around the circumference.

3. Auditory ossicle prosthesis according to claim 1, **characterised by** precisely three spikes (29') arranged on the outer edge (28) in such a manner that they are offset by 120° relative to one another around the circumference.

4. Auditory ossicle prosthesis according to claim 1, **characterised by** precisely four spikes (29") arranged in pairs opposite one another on the outer edge (28) in such a manner that they are offset by 90° relative to one another around the circumference.

5. Auditory ossicle prosthesis according to one of the preceding claims, **characterised in that** the spikes (19; 29; 29'; 29") are triangular, the base of the triangle being rigidly connected to the outer edge (18; 28) of the inner surface of the receiving space (15; 25) and the vertex of the triangle opposite the base projecting away from the inner surface.

6. Auditory ossicle prosthesis according to one of the preceding claims, **characterised in that** the elongated prosthesis body (33; 43; 53) has at least one joint (37; 47; 57).

7. Auditory ossicle prosthesis according to claim 6, **characterised in that** the joint (57) includes a ball (57') mounted at the end of the elongated prosthesis body (53) directed towards the second coupling element (52), a sleeve (57") covering the ball (57') on its side directed away from the second coupling element (52) and a recess (57"') provided in the side directed towards the second coupling element (52) and acting as a socket for the ball (57').

8. Auditory ossicle prosthesis according to claim 7, **characterised in that** the sleeve (57") is formed from a plastic casting compound.

9. Auditory ossicle prosthesis according to one of the preceding claims, **characterised in that** the second coupling element (32; 42; 52; 62) is provided on the inner surface of the receiving space (25) forming an axial continuation of the elongated prosthesis body (33; 43; 53; 63) with a filling portion (36; 46; 56; 66) projecting away from the elongated prosthesis body (33; 43; 53; 63) into the receiving space (25), bearing against the stapes in the implanted state of the auditory ossicle prosthesis (30; 40; 50; 60) and preventing or minimising the formation of a cavity between the stapes and the inner surface of the receiving space (25) forming an axial continuation of the elongated prosthesis body (33; 43; 53; 63).

10. Auditory ossicle prosthesis according to claim 9, **characterised in that** the filling portion (36; 46; 56; 66) is spherical or ellipsoidal and is arranged symmetrically relative to the extended axis of the elongated prosthesis body (33; 43; 53; 63).

11. Auditory ossicle prosthesis according to claim 9, **characterised in that** the filling portion is conical and is arranged symmetrically relative to the extended axis of the elongated prosthesis body, the vertex of the cone projecting away from the elongated prosthesis body into the receiving space.

12. Auditory ossicle prosthesis according to claim 9, **characterised in that** the filling portion is cylindrical and is arranged symmetrically relative to the extended axis of the elongated prosthesis body, the cylinder projecting away from the elongated prosthesis body into the receiving space.

13. Auditory ossicle prosthesis according to claim 9, **characterised in that** the filling portion is piston-shaped and is arranged symmetrically relative to the extended axis of the elongated prosthesis body, the piston shaft supporting the piston body projecting away from the elongated prosthesis body into the receiving space.

14. Auditory ossicle prosthesis according to claim 13, **characterised in that** the piston body has a concave or flat bearing surface directed into the receiving space.

15. Auditory ossicle prosthesis according to claim 13, **characterised in that** the piston body has a convex bearing surface directed into the receiving space.

## Revendications

1. Prothèse d'osselets (10 ; 20 ; 30 ; 40 ; 50 ; 60) qui est réalisée pour remplacer ou ponter au moins un élément de la chaîne d'osselets humaine, comportant un corps de prothèse oblong (13 ; 23 ; 33 ; 43 ; 53 ; 63) transmetteur du son qui présente à l'une de ses extrémités un premier élément de couplage (11 ; 21 ; 31 ; 41 ; 61) qui est configuré sous la forme d'une plaque de tête (11 ; 21 ; 31 ; 41) destinée à l'appui de la prothèse contre le tympan ou sous la forme d'un clip destiné à la liaison mécanique avec un organe de la chaîne d'osselets ou encore sous la forme d'un élément raccord (61) destiné à la liaison conductrice du son avec un embout d'actionneur d'un implant auditif actif,
dans laquelle
le corps de prothèse oblong (13 ; 23 ; 33 ; 43 ; 53 ; 63) porte, à son autre extrémité, un second élément de couplage (12 ; 22 ; 32 ; 42 ; 52 ; 62) configuré sous la forme d'un clip (12) pourvu de plusieurs languettes (12a) et de plusieurs fentes (12b) latérales en alternance mutuelle ou sous la forme d'une cloche (22 ; 32 ; 42 ; 52 ; 62) latéralement fendue plusieurs fois pour une liaison mécanique de la prothèse avec un étrier ou avec une tête d'étrier, qui comprend une ouverture d'accès (14 ; 24) dans un espace de logement (15 ; 25) avec une surface intérieure dans le prolongement axial du corps de prothèse oblong (13 ; 23 ; 33 ; 43 ; 53 ; 63), qui est délimitée par un bord extérieur (18 ; 28) périphérique interrompu plusieurs fois,
**caractérisée en ce que**
sur le bord extérieur (18 ; 28) de la surface intérieure de l'espace de logement (15 ; 25), le second élément de couplage (12 ; 22 ; 32 ; 42 ; 52 ; 62) comprend au moins deux pointes (19 ; 29 ; 29' ; 29") agencées en répartition sur la périphérie du bord extérieur (18 ; 28), qui s'étendent dans une direction parallèle au prolongement axial du corps de prothèse oblong (13 ; 23 ; 33 ; 43 ; 53 ; 63) et qui, dans l'état implanté de la prothèse d'osselets (10 ; 20 ; 30 ; 40 ; 50 ; 60), viennent s'engager dans l'étrier pour y assurer un maintien sûr du second élément de couplage (12 ; 22 ; 32 ; 42 ; 52 ; 62).

2. Prothèse d'osselets selon la revendication 1, **caractérisée en ce qu'**il existe précisément deux pointes (19 ; 29) qui se font face sur le bord extérieur (28) en étant décalées de 180° sur la périphérie.

3. Prothèse d'osselets selon la revendication 1, **caractérisée en ce qu'**il existe précisément trois pointes (29') qui sont agencées les unes par rapport aux autres sur le bord extérieur (28) en étant décalées de 120° sur la périphérie.

4. Prothèse d'osselets selon la revendication 1, **caractérisée en ce qu'**il existe précisément quatre pointes (29") qui sont agencées les unes par rapport aux autres sur le bord extérieur (28) en étant décalées de 90° sur la périphérie et en se faisant face par paire.

5. Prothèse d'osselets selon l'une des revendications précédentes, **caractérisée en ce que** les pointes (19 ; 29 ; 29' ; 29") sont de forme triangulaire, une base du triangle étant fermement reliée au bord extérieur (18 ; 28) de la surface intérieure de l'espace de logement (15 ; 25), et une pointe opposée à la base du triangle étant dirigée en éloignement de la surface intérieure.

6. Prothèse d'osselets selon l'une des revendications précédentes, **caractérisée en ce que** le corps de prothèse oblong (33 ; 43 ; 53) comprend au moins une articulation (37 ; 47 ; 57).

7. Prothèse d'osselets selon la revendication 6, **caractérisée en ce que** l'articulation (57) comprend une bille (57') montée à son extrémité du corps de prothèse oblong (53) tournée vers le second élément de couplage (52), une enveloppe (57") recouvrant la bille (57') sur son côté détourné du second élément de couplage (52), ainsi qu'un renfoncement (57"') dans le côté tourné vers le second élément de couplage (52), qui fait office de cavité articulaire pour la bille (57').

8. Prothèse d'osselets selon la revendication 7, **caractérisée en ce que** l'enveloppe (57") est constituée d'un scellement de matière plastique.

9. Prothèse d'osselets selon l'une des revendications précédentes, **caractérisée en ce que** sur la surface intérieure de l'espace de logement (25), en prolongement axial du corps de prothèse oblong (33 ; 43 ; 53 ; 63), le second élément de couplage (32 ; 42 ; 52 ; 62) comprend une portion de rembourrage (36 ; 46 ; 56 ; 66) dirigée en éloignement du corps de prothèse oblong (33 ; 43 ; 53 ; 63) et pénétrant dans l'espace de logement (25), portion qui, dans l'état implanté de la prothèse d'osselets (30 ; 40 ; 50 ; 60), s'appuie contre l'étrier et empêche ou minimise la réalisation d'une cavité entre l'étrier et la surface intérieure de l'espace de logement (25) en prolongement axial du corps de prothèse oblong (33 ; 43 ; 53 ; 63).

10. Prothèse d'osselets selon la revendication 9, **caractérisée en ce que** la portion de rembourrage (36 ; 46 ; 56 ; 66) est configurée de façon sphérique ou ellipsoïdale et est agencée symétriquement par rapport au prolongement de l'axe du corps de prothèse oblong (33 ; 43 ; 53 ; 63).

11. Prothèse d'osselets selon la revendication 9, **caractérisée en ce que** la portion de rembourrage est configurée sous forme conique et est agencée symétriquement par rapport au prolongement de l'axe du corps de prothèse oblong, une pointe du cône étant dirigée en éloignement du corps de prothèse oblong et pénétrant dans l'espace de logement.

12. Prothèse d'osselets selon la revendication 9, **caractérisée en ce que** la portion de rembourrage est configurée sous forme cylindrique et est agencée symétriquement par rapport au prolongement de l'axe du corps de prothèse oblong, un cylindre étant dirigé en éloignement du corps de prothèse oblong et pénétrant dans l'espace de logement.

13. Prothèse d'osselets selon la revendication 9, **caractérisée en ce que** la portion de rembourrage est configurée sous forme de poinçon et est agencée symétriquement par rapport au prolongement de l'axe du corps de prothèse oblong, une tige de poinçon portant un corps de poinçon étant dirigée en éloignement du corps de prothèse oblong et pénétrant dans l'espace de logement.

14. Prothèse d'osselets selon la revendication 13, **caractérisée en ce que** le corps de poinçon comprend une surface de support concave ou plane dirigée dans l'espace de logement.

15. Prothèse d'osselets selon la revendication 13, **caractérisée en ce que** le corps de poinçon comprend une surface de support convexe dirigée dans l'espace de logement.
